# EUROPEAN PATENT APPLICATION

(11) **EP 1 034 795 A2**
(43) Date of publication of application: **13.09.2000**
(21) Application number: 00200819.1
(22) Date of filing: 08.07.1993
(51) Int. Cl.: A61K 47/48

(54) **Conjugates of lipids and biologically active compounds**

(30) Priority: 09.07.1992 US 911209
(62) Divisional of application: 93917054.4
(71) Applicant: THE STATE OF OREGON acting by and through THE STATE BOARD OF HIGHER EDUCATION ON BEHALF OF OREGON HEALTH SCIENCES UNIVERSITY, Portland, OR 97201-3098 (US)
(72) Inventor: Yatvin, Milton B., Portland, OR 97201 (US); Stowell, Michael H. B., Cambridge CB2 2QH (GB); Malkovsky, Miroslav, Madison, WI 53705 (US); McClard, Ronald W., Portland, OR 97215 (US); Parks, David Wei-Sun, Seattle, WA 98115 (US); Witt, John F., Portland, OR 97206 (US)
(74) Representative: Williams, Richard Andrew Norman

(57) **Abstract**

Biologically active compounds are coupled to polar lipids to provide conjugates. The conjugates have specificity for subcellular membranes by virtue of the physical and chemical properties of the lipid components. The conjugates can accumulate specifically in the desired subcellular membrane and the nature of the coupling of the biologically active compounds to the lipids is such that the compounds may be released in free form into a desired subcellular compartment.

## Description

### BACKGROUND OF THE INVENTION

### 1. Field of the Invention

This invention relates to methods of facilitating the entry of biologically-active compounds into cells and for targeting such compounds to specific organelles within the cell. The invention specifically provides compositions of matter and pharmaceutical embodiments of such compositions comprising conjugates of such biologically-active compounds covalently linked to polar lipid carrier molecules. Particular embodiments of such conjugates comprise antiviral or antineoplastic drugs for facilitating entry of such drugs into cells at pharmacokinetically useful levels and also for targeting such drugs to specific organelles within the cell. Using the methods of the invention, intracellular drug concentrations may reach levels which are orders of magnitude higher than those achieved otherwise. The invention also provides polar lipid conjugates of antigenically-active peptides for facilitating the entry of peptides into cells and specific organelles within the cell for the specific production of immunological responses by such peptides. As such, this invention provides improved methods for vaccine production and *in vivo* vaccination against pathogenic microorganisms, and for alleviating autoimmune disease and ameliorating tissue and organ transplant rejection.

### 2. Background of the Related Art

A major goal in the pharmacological arts has been the development of methods and compositions to facilitate the specific delivery of therapeutic and other agents to the appropriate cells and tissues that would benefit from such treatment, and the avoidance of the general physiological effects of the inappropriate delivery of such agents to other cells or tissues of the body. The most common example of the need for such specificity is in the field of antineoplastic therapy, in which the amount of a variety of anti-neoplastic agents that can be safely administered to a patient is limited by their cytotoxic effects.

In addition, certain tissues have proven inaccessible or of limited accessibility to drug administration using conventional techniques. A particular example is the brain, which is protected by the "blood-brain barrier", which limits the amount of certain pharmacological substances that can be delivered to the brain *via* the bloodstream. Although the existence of the blood-brain barrier is thought to be beneficial in protecting the brain from environmental toxins, pathogens and other toxic substances, this structure also limits the effectiveness of clinical intervention in cases of malignancy (*e.g*., neuroblastoma) or infection (*e.g*., meningitis).

Finally, it is recognized in the medical arts that certain subcellular organelles are the sites of pharmacological action of certain drugs or are involved in the biological response to certain stimuli. Specific delivery of diagnostic or therapeutic compounds to such intracellular organelles is thus desireable to increase the specificity and effectiveness of such clinical diagnostic or therapeutic techniques.

### A. Drug Targeting

It is desirable to increase the efficiency and specificity of administration of a therapeutic agent to the cells of the relevant tissues in a variety of pathological states. This is particularly important as relates to antiviral or antineoplastic drugs. These drugs typically have pleiotropic antibiotic and cytotoxic effects that damage or destroy uninfected or untransformed cells as well as virally infected or malignant cells. Thus, an efficient delivery system which would enable the delivery of such drugs specifically to infected or malignant cells would increase the efficacy of treatment and reduce the associated "side effects" of such drug treatments, and also serve to reuce morbidity and mortality associated with clinical administration of such drugs.

Numerous methods for enhancing the cytotoxic activity and the specificity of antiviral and antineoplastic drug action have been proposed. One method, receptor taregeting, involves linking the therapeutic agent to a ligand which has an affinity for a receptor expressed on the desired target cell surface. Using this approach, an antiviral or antineoplastic drug is intended to adhere to the target cell following formation of a ligand-receptor complex on the cell surface. Entry into the cell could then follow as the result of internalization of ligand-receptor complexes. Following internalization, the antiviral or antineoplastic drug may then exert its therapeutic effects directly on the cell.

One limitation of the receptor targeting approach lies in the fact that there are only a finite number of receptors on the surface of target cells. It has been estimated that the maximum number of receptors on a cell is approximately one million (Darnell *et al*., 1986, *Molecular Cell Biology*, 2d ed., W.H. Freeman: New York, 1990). This estimate predicts that there may be a maximum one million drug-conjugated ligand-receptor complexes on any given cell. Since not all of the ligand-receptor complexes may be internalized, and any given ligand-receptor system may express many-fold fewer receptors on a given cell surface, the efficacy of intracellular drug delivery using this approach is uncertain. Other known intracellular ligand-receptor complexes (such as the steroid hormone receptor) express as few as ten thousand hormone molecules per cell. *Id.* Thus, the ligand-receptor approach is plagued by a number of biological limitations.

Other methods of delivering therapeutic agents at concentrations higher than those achievable through the receptor targeting process include the use of lipid conjugates that have selective affinities for specific biological membranes. These methods have met with little success. (*see, for example*, Remy *et al*., 1962, J. Org. Chem. 27: 2491-2500; Mukhergee & Heidelberger, 1962, Cancer Res. 22: 815-22; Brewster *et al*., 1985, J. Pharm. Sci. 77: 981-985).

Liposomes have also been used to attempt cell targeting. Rahman *et al*., 1982, Life Sci. 31: 2061-71 found that liposomes which contained galactolipid as part of the lipid appeared to have a higher affinity for parenchymal cells than liposomes which lacked galactolipid. To date, however, efficient or specific drug delivery has not been predictably achieved using drug-encapsulted liposomes. There remains a need for the development of a cell- or organelle-targeting drug delivery system.

### B. Targeting of Immunologically-Active Peptides and Vaccine Production

Vertebrate immunological responses to antigenic stimuli depend on a phenomenon called presentation of antigens. Presentation embodies the appearance of antigenic epitopes on the cell surface of antigen-presenting cells of the immune system in association with major histocompatibility complex (MHC) proteins. The MHC proteins are divided into 2 classes (I & II) whereby each class presents antigens derived from different sources. The two types of immune response corresponding to presentation *via* the two types of MHC molecules are said to be MHC class I or class II restricted. Both MHC class I and class II restricted immunity requires presentation of peptide antigens in association with MHC molecules (*see*, Abbas, Lichtman & Pober, 1991, *Cellular and Molecular Immunology* (W.B. Saunders Co.: Philadelphia), pp. 116-136 for review).

In the class I-restricted immune response, MHC class I molecules are associated with peptide antigens derived from proteins made intracellularly. Such proteins include proteins encoded by viruses and other intracellular pathogens. These proteins are degraded in the cytoplasm of infected cells, and the peptide products of this degradation transferred into the endoplasmic reticulum (ER) *via* the action of peptide-specific transporter molecules located in the ER membrane (*see*, Elliott *et al*., 1990, Nature 348: 195-197; Parham, 1990, Nature 348: 674-675). Nascent MHC class I molecules synthesized in the ER are assembled into functional presenting proteins only in the presence of the appropriate peptide antigen. Fully assembled MHC class I complexes are then transported through the Golgi apparatus to the cell surface, where the antigen presenting complex can activate a cellular (T-cell mediated) immune response by interacting with CD8⁺ cytotoxic T-cells (*see*, Falk *et al*., 1990, Nature 348: 248-251; Falk *et al*., 1991, Nature 351: 290-296).

Alternatively, in the MHC class II restricted immune response, extracellular antigens (including free-living pathogens or protein components thereof) are engulfed by cells of the immune system (such as macrophages) by endocytosis, and transferred to the endosomal (lysosomal) compartment for degradation. Peptide products of such degradation may then associate with MHC class II molecules (which molecules lack the requirement of peptide association for cell-surface expression; *see*, Germain & Hendrix, 1991, Nature 353: 134-139) and appear on the cell surface (*see*, Sadegh-Nasseri & Germain, 1991, Nature 353: 167-170; Lanzavecchia *et al*., 1992, Nature 357: 249-252). The MHC class II antigen-presenting pathway leads to the induction of a humoral (antibody-dependent) immune response and the activation of CD4⁺ T-helper cells.

In the preparation and use of vaccines to provoke immunity to pathogenic organisms, the appropriate MHC restriction is achieved using alternative strategies (*see*, Abbas, Lichtman & Pober, 1991, *Cellular and Molecular Immunology* (W.B. Saunders Co.: Philadelphia), p. 315 *et seq.* for review). MHC class I restricted immunity requires the use of attenuated pathogenic organisms (usually viruses) which non-productively infect host cells. The protein antigens of the pathogenic organism is degraded intracellularly, and the peptide antigens produced transduced to the ER and assembled into functional MHC class I presentation complexes. Presentation of antigen to cytotoxic T-cells results in cellular immunity against the pathogenic microorganism.

Vaccination using the MHC class II-restricted route involves inoculation with inactivated (*e.g.,* chemically inactivated) pathogens, which are then engulfed by macrophages and lysosomally degraded intracellularly. Peptide antigens associated with the appropriate MHC class II complex are then presented to T-helper cells, which cells release cytokines and other immune system stimulating factors, which activate antibody-producing B cells specific for the peptide antigen presented.

Both routes to producing immunity to pathogenic organisms require degradation and selection of the appropriate peptide antigen by the cells of an animals immune system *in vivo.* This allows for variability in the efficacy of production of the immune response, and in the case of the use of attenuated viruses, the possibility for reversion to pathogenicity (with the result that the vaccine causes the disease it was meant to forestall). There is a need, therefore, for developing methods to efficiently deliver peptide antigens directly to cells of the immune system for presentation to T-cells in association with the appropriately restricted MHC complex.

Similarly, autoimmune disease is related to the presentation and immunological recognition of peptide antigens derived from endogenous cellular proteins (called self-antigens; *see,* Jardetzky *et al.,* 1991, Nature 353: 326-329; Faustman *et al*., 1991, Science 254: 1756-1771). Self-antigens may be presented by either the MHC class I or class II restricted pathway, resulting in either cellular or humoral autoimmunity, or both. There is a need to develop methods and reagents to block presentation of self-antigens, thereby ameliorating or preventing the onset of progression of autoimmune disease.

In addition, tissue or organ transplant rejection is mediated by both MHC class I and class II restricted immune response. Non-self antigens are processed and recognized by the immune system of the transplant recipient, causing an immunological attack on the transplant resulting in its rejection by the host. Current methodologies of inhibiting transplant rejection involve suppressing the immune system indiscriminately with drugs such as cyclosporin A. These methods leave the host immune-compromised and at risk for adventitious infection by pathogens. There is a need for methods of selectively blocking host MHC restricted immune response against tissue and organ transplants which do not result in general immune suppression.

The use of peptide antigens as immunogens has been attempted in the prior art, with limited success *in vivo*.
Hopp, 1984, Mol. Immunol. 21: 13-16 disclosed the use of a synthetic hepatitis viral antigen acylated with a fatty acid moiety as an *in vivo* immunogen.
Neurath *et al*., 1984, J. Gen. Virol. 65: 1009-1014 utilize hepatitis surface antigen-derived peptide immunogens that are chemically fixed to liposomes *in vitro*.
Deres *et al*., 1989, Nature 342: 561-564 disclose the use of influenza peptide epitopes chemically linked to lipoprotein adjuvants as immunogens *in vivo.*
Seifert *et al.,* 1990, J. Biochem. 267: 795-802 teach the use of lipoprotein-derived synthetic antigens for activating human neutrophils *in vitro*.
Brynestad *et al.,* 1990, J. Virol. 64: 680-685 use palmitoylated peptide antigens derived from herpes simplex virus glycoprotein D as immunogens *in vivo.*
Wiesmüller *et al.,* 1991, Immunology 72: 109-113 demonstrate that synthetic lipoprotein analogues stimulate cytokine release and activate B cells and macrophages *in vitro.*
Frisch *et al.,* 1991, Eur. J. Immunol. 21: 185-193 describe the use of a histone H3 derived, hexapeptide antigen covalently attached to phosphatidylethanolamine and encapsulated into liposomes for immunizing mice *in vivo.*

Peptides have also been used for blocking an immune response.
Vandenbark *et al.,* 1989, Nature 341: 841-844 demonstrate that a peptide derived from the T-cell receptor Vβ8 chain can be used to block experimentally-induced autoimmune encephalitis (EAE) *in vivo.*
Lamont *et al.,* 1990, J. Immunol. 144: 2493-2498 disclose peptide MHC class II inhibitors of antigen presentation.
Guéry *et al.,* 1992, J. Exp. Med. 175: 1345-1352 demonstrate inhibition of antigen presentation *in vivo* using a blocking peptide antigen.
DeMagriatis *et al.,* 1992, Cell 68: 625-634 show the use of an influenza peptide to block T-cell mediated immunity *in vitro.*

### SUMMARY OF THE INVENTION

The present invention is directed to an improved method for delivering biologically-active compounds to cells and cellular organelles *in vivo* and *in vitro.* This delivery system achieves such specific delivery of biologically-active compounds through conjugating the compounds with a polar lipid carrier. This invention has the specific advantage of facilitating the entry of such compounds into cells *via* the polar lipid carrier, achieving effective intracellular concentration of such compounds more efficiently and with more specificity than conventional delivery systems.

The invention provides compositions of matter comprising a biologically-active compound covalently linked to a polar lipid carrier molecule. Preferred embodiments also comprise a spacer molecule having two linker functional groups, wherein the spacer has a first end and a second end and wherein the polar lipid is attached to the first end of the spacer through a first linker functional group and the biologically-active compound is attached to the second end of the spacer through a second linker functional group. In preferred embodiments of the invention, the biologically-active compound is a peptide, most preferably an antigenically active peptide. In other preferred embodiments, the biologically-active compound is a drug, most preferably an antiviral or antineoplastic drug. Preferred polar lipids include but are not limited to sphingosine, ceramide, phosphatidyl choline, phosphatidyl glycerol, phosphatidyl ethanolamine, phosphatidyl inositol, phosphatidyl serine, cardiolipin and phosphatidic acid.

The invention also provides compositions of matter comprising a biologically-active compound covalently linked to a polar lipid carrier molecule *via* a spacer molecule wherein the spacer allows the biologically-active compound to act without being released at an intracellular site. In these embodiments of the invention, the first linker functional group attached to the first end of the spacer is characterized as "strong" and the second linker functional group attached to the second end of the spacer is characterized as "weak", with reference to the propensity of the covalent bonds between each end of the spacer molecule to be broken.

In other embodiments of the compositions of matter of the invention, the spacer allows the facilitated hydrolytic release of the biologically-active compound at an intracellular site. Other embodiments of the spacer facilitate the enzymatic release of the biologically-active compound at an intracellular site.

In another embodiment of this aspect of the invention, the spacer molecule is a peptide of formula (amino acid)ₙ, wherein n is an integer between 2 and 25, preferably wherein the peptide comprises a polymer of a particular amino acid.

In other embodiments of the compositions of matter of the invention, the biologically-active compound of the invention has a first functional linker group, and a polar lipid carrier has a second functional linker group, and the compound is directly covalently linked to the polar lipid carrier by a chemical bond between the first and second functional linker groups. In preferred embodiments, each of the first and second functional linker groups is a hydroxyl group, a primary or secondary amino group, a phosphate group or substituted derivatives thereof or a carboxylic acid group.

In another aspect of the invention is provided compositions of matter comprising a drug covalently linked to a polar lipid carrier molecule. Preferred embodiments also comprise a spacer molecule having two linker functional groups, wherein the spacer has a first end and a second end and wherein the polar lipid is attached to the first end of the spacer through a first linker functional group and the drug is attached to the second end of the spacer through a second linker functional group. In preferred embodiments of the invention, the drug is an antiviral or antineoplastic drug, most preferably azidothymidine or methotrexate. Preferred polar lipids include but are not limited to sphingosine, ceramide, phosphatidyl choline, phosphatidyl glycerol, phosphatidyl ethanolamine, phosphatidyl inositol, phosphatidyl serine, cardiolipin and phosphatidic acid.

The invention also provides compositions of matter comprising an antiviral or antineoplastic drug covalently linked to a polar lipid carrier molecule *via* a spacer molecule wherein the spacer allows the drug to act without being released at an intracellular site. In these embodiments of the invention, the first linker functional group attached to the first end of the spacer is characterized as "strong" and the second linker functional group attached to the second end of the spacer is characterized as "weak", with reference to the propensity of the covalent bonds between each end of the spacer molecule to be broken.

In other embodiments of the compositions of matter of the invention, the spacer allows the facilitated hydrolytic release of the antiviral or antineoplastic drug at an intracellular site. Other embodiments of the spacer facilitate the enzymatic release of the antiviral or antineoplastic drugs of the invention at an intracellular site.

In still further embodiments of the compositions of matter of the invention, the antiviral or antineoplastic drug of the invention has a first functional linker group, and a polar lipid carrier has a second functional linker group, and the drug is directly covalently linked to the polar lipid carrier by a chemical bond between the first and second functional linker groups. In preferred embodiments, each of the first and second functional linker groups is a hydroxyl group, a primary or secondary amino group, a phosphate group or substituted derivatives thereof or a carboxylic acid group.

In another embodiment of this aspect of the invention, the spacer molecule is a peptide of formula (amino acid)ₙ, wherein n is an integer between 2 and 25, preferably wherein the peptide comprises a polymer of a particular amino acid.

Preferred embodiments of this aspect of the invention include compositions of matter that are 1-phenyl-2-(alanylalanyl)amino-3-hydroxyl-pentanoylvalinylvalinylglycylglycylglycylgylcyl ceremide ester, 3'-azido-3'-deoxythymidine monophosphate-[tri-β-hydroxypropionylester]-O^{x}-ceremide ester, 3'-azido-3'-deoxythymidine monophosphate-glycylglycylglycylgylcyl ceremide ester, N-[1-(3'-azido-3'-deoxythymidinemonophosphate)amninohexanoyl] sphingosine amide, 1-phenyl-2-(alanylalanyl)amino-3-hydroxyl-pentanoyl-valinylvalinyl sphingosine amide, 3'-azido-3'-deoxythymidine monophosphate-O^{x}-ceremide ester, 5-fluorouridine deoxyribosemonophosphate-O^{x}-ceremide ester, phosphatidylcholine-3'-azido-3'-deoxythymidinemonophosphate,phosphatidylglycerol-3'-azido-3'-deoxythymidine monophosphate, *N-*methotrexate ceremide, and phosphatidylethanolamine-3'-azido-3'-deoxythymidine monophosphate.

As disclosed herein, the invention comprehends a lipid-drug conjugate wherein the lipid will selectively associate with certain biological membranes, and thereby facilitate entry of the drug into cells and cellular organelles. The spacer component of the conjugates of the invention will preferably act to release the drug from the lipid, target the conjugate to the cell, incorporate the drug into a viral envelope, or perform other functions to maximize the effectiveness of the drug.

This type of conjugate has numerous advantages. First, the drug-polar lipid conjugates of the invention will promote the intracellular entry of a variety of potentially useful antiviral and antineoplastic drugs at pharmokinetical rates not currently attainable. Second, the range of targeted cell types is not limited *per se* by particular, limited biological properties of the cell such as the number and type of specific receptor molecules expressed on the cell surface, as are receptor-specific drug targeting methods. Third, in contrast to traditional attempts to simply target drugs to specific cells, this method may target drugs to specific intracellular organelles and other intracellular compartments. Fourth, the compositions of matter of the invention incorporate a variable spacer region that may allow pharmacologically-relevant rates of drug release from polar lipid carrier molecules to be engineered into the compositions of the invention, thereby increasing their clinical efficacy and usefulness. Thus, time-dependent drug release and specific drug release in cells expressing the appropriate degradative enzymes are a unique possibility using the drug-lipid conjugates of the invention. Finally, the conjugates of the invention can be combined with other drug delivery approaches to further increase specificity and to take advantage of useful advances in the art. One example for antiviral therapy would involve incorporating the conjugates of the invention into the viral envelope, thereby directly modifying its lipid composition and influencing viral infectivity.

The present invention is also directed to methods for eliciting or inhibiting an immune response in an animal, preferably a human. The invention provides reagents comprising antigenically-active peptides covalently linked to polar lipid carrier molecules. Conjugation of the antigenically-active peptides to the polar lipid can be mediated by a spacer moiety. The choice of polar lipid conjugated to the antigenically-active peptides of the invention will influence the intracellular site to which such peptide/lipid conjugates are targeted. Methods for using the reagents of the invention are also provided.

This invention has the specific advantage of facilitating the entry of antigenically-active peptides into cells of the immune system *via* a polar lipid carrier, allowing introduction of such peptides into cells in the absence of intracellular production of the peptides and without requiring endocytosis of such peptides into the degradative compartment of such cells. As disclosed herein, the invention comprehends a polar lipid/peptide conjugate wherein the polar lipid will selectively associate with certain biological membranes, and facilitate intracellular localization of the peptides therein.

The polar lipid may also be conjugated to the antigenically-active peptide through use of a spacer, which may act to release the peptide from the lipid, target the conjugate to the proper intracellular compartment, or perform other functions to maximize the effectiveness of immunological processing by the cell.

This type of conjugate is advantageous for a number of reasons. First, the ability of this invention to allow the entry of antigenically-active peptides into cells at pharmokinetic rates eliminates the requirement using traditional vaccination methods for intracellular synthesis of viral peptide antigens destined for presentation *via* the major histocompatibility complex class I antigen presentation pathway. Second, for antigens presented *via* the major histocompatibility complex class II antigen presentation pathway, the specific antigenic epitope can be delivered to the lysosomal compartment of the cell for association with nascent MHC class II molecules without the need for intracellular proteolysis of the cognate protein of the peptide antigen. Third, the reagents of the invention may incorporate a spacer region that can be varied and thereby allow an immunologically-relevant rate of antigen release in antigen-presenting cells.

In a first embodiment of this aspect, this invention provides a composition of matter comprising a peptide, a polar lipid carrier, two linker functional groups and a spacer, wherein the spacer has a first end and a second end and wherein the polar lipid is attached to the first end of the spacer through a first linker functional group and the peptide is attached to the second end of the spacer through a second linker functional group. In a preferred embodiment, the peptide is an antigenically active peptide. In another preferred embodiment, the spacer allows the peptide to act without being released at an intracellular site. In this embodiment of the invention, the covalent attachment of the first linker functional group to the first end of the spacer is weak and the covalent attachment of the second linker functional group to the second end of the spacer is strong; alternatively, both covalent attachments are strong. In other embodiments, the spacer facilitates hydrolytic or enzymatic release of the peptide at an intracellular site. In this embodiment, the covalent attachment of the first linker functional group to the first end of the spacer is strong and the second linker functional group attached to the second end of the spacer is weak. Preferred polar lipids include sphingosine, ceramide, phosphatidyl choline, phosphatidyl glycerol, phosphatidyl ethanolamine, phosphatidyl inositol, phosphatidyl serine, cardiolipin and phosphatidic acid.

A second embodiment of this aspect of the invention provides a composition of matter comprising a peptide having a first functional linker group, and a polar lipid carrier having a second functional linker group, wherein the peptide is covalently linked to the polar lipid carrier by a chemical bond between the first and second functional linker groups. In a preferred embodiment, the peptide is an antigenically active peptide. Preferred first and second functional linker groups each independently include a hydroxyl group, a primary or secondary amino group, a phosphate group or substituted derivatives thereof and a carboxylic acid group. Preferred polar lipids include sphingosine, ceramide, phosphatidyl choline, phosphatidyl glycerol, phosphatidyl ethanolamine, phosphatidyl inositol, phosphatidyl serine, cardiolipin and phosphatidic acid.

In another embodiment of this aspect of the invention, the spacer molecule is a peptide of formula (amino acid)ₙ, wherein n is an integer between 2 and 25, preferably wherein the peptide comprises a polymer of a particular amino acid.

The invention provides a method of immunizing an animal against a pathogenic microorganism, comprising the step of inoculating the animal with a reagent that is a composition of matter of the invention in a pharmaceutically acceptable carrier in an amount sufficient to elicit an immunological response in the animal. The preferred animal is a human being.

The invention also provides a method of alleviating autoimmune disease in an animal, comprising the step of inoculating the animal with a reagent that is a composition of matter of the invention in a pharmaceutically acceptable carrier in an amount sufficient to inhibit the autoimmune response in the animal. The preferred animal is a human being.

The invention further provides a method for preventing tissue or organ transplant rejection in an animal, comprising the step of inoculating the animal with a reagent that is a composition of matter of the invention in a pharmaceutically acceptable carrier in an amount sufficient to inhibit transplant rejection in the animal. The preferred animal is a human being.

Specific preferred embodiments of the present invention will become evident from the following more detailed description of certain preferred embodiments and the claims.

### BRIEF DESCRIPTION OF THE DRAWINGS

Figure 1 depicts the synthetic scheme put forth in Example 1.
Figure 1A depicts the synthetic scheme put forth in Example 1A.
Figure 2 depicts the synthetic scheme put forth in Example 2.
Figure 3 depicts the synthetic scheme put forth in Example 3.
Figure 3A depicts the synthetic scheme put forth in Example 3A.
Figure 4 depicts the synthetic scheme put forth in Example 4.
Figure 4A depicts the synthetic scheme put forth in Example 4A.
Figure 5 depicts the synthetic scheme put forth in Example 5.
Figure 5A depicts the synthetic scheme put forth in Example 5A.
Figure 6 depicts the synthetic scheme put forth in Example 6.
Figure 7 depicts the synthetic scheme put forth in Example 7.
Figure 8 depicts the synthetic scheme put forth in Example 8.
Figure 9 depicts the synthetic scheme put forth in Example 9.
Figure 10 depicts the synthetic scheme put forth in Example 10.
Figure 11 depicts the synthetic scheme put forth in Example 11.
Figure 12 depicts the synthetic scheme put forth in Example 12.

### DETAILED DESCRIPTION OF THE PREFERRED EMBODIMENTS

The present invention provides compositions of matter and methods for facilitating the entry into cells of biologically-active compounds. For the purposes of this invention, the term "biologically-active compound" is intended to encompass all naturally-occurring or synthetic compounds capable of eliciting a biological response or having an effect, either beneficial or cytotoxic, on biological systems, particularly cells and cellular organelles. These compounds are intended to include but are not limited to all varieties of drugs, particularly antimicrobial drugs such as antiviral, antibacterial, fungicidal and antiprotozoal, especially antiplasmodial drugs, and antineoplastic drugs, particularly methotrexate and 5-fluorouracil and other antineoplastic drugs, as well as peptides including peptide hormones and peptide fragments of proteins, particularly pathogenic and microbially-derived proteins, useful, for example, as vaccines and otherwise. A particular example herein disclosed useful in the practice of the invention is HIV1 protease inhibitor (identified as compound 8; Dreyer *et al.,* 1986, Proc. Natl. Acad. Sci. USA 86: 9752-56).

The compositions of matter provided by the invention comprise the biologically-active compounds of the invention covalently linked to a polar lipid carrier. A polar lipid carrier, as defined herein is intended to mean any polar lipid having an affinity for, or capable of crossing, a biological membrane, including but not limited to sphingosine, ceramide, phosphatidyl choline, phosphatidyl glycerol, phosphatidyl ethanolamine, phosphatidyl inositol, phosphatidyl serine, cardiolipin, phosphatidic acid, sphingomyelin and other sphingolipids, as these terms are understood in the art (*see,* Lehninger, *Biochemistry,* 2d ed., Chapters 11 & 24, Worth Publishers: New York, 1975).

The compositions of matter of the invention may be further comprised of a spacer moiety comprising a first end and a second end, each end of the spacer having a functional linking group. For the purposes of this invention, the term "spacer" or "spacer moiety" is intended to encompass any chemical entity that links the biologically-active compound and the polar lipid. Such spacer moieties may be designed to facilitate the attachment of the conjugates of the invention to a target cell, or to facilitate, influence, modulate or regulate the release of the biologically-active compound at the desired target site. Such spacers may also facilitate enzymatic release at certain intracellular sites. Spacer groups, as described herein, include, but are not limited to aminohexanoic acid, polyglycine, polyamides, polyethylenes, and short functionalized polymers having a carbon backbone which is from one to about twelve carbon molecules in length. Particularly preferred embodiments of such spacer moieties comprise peptides of formula (amino acid)ₙ, wherein n is an integer between 2 and 25 and the peptide is a polymer of a particular amino acid.

The term "linker functional group" is defined herein as any functional group for covalently binding the polar lipid carrier or biologically-active agent to the spacer group. These groups can be designated either "weak" or "strong" based on the stability of the covalent bond which the linker functional group will form between the spacer and either the polar lipid carrier or the biologically-active compound. The weak functionalities include, but are not limited to phosphoramide, phosphoester, carbonate, amide, carboxyl-phosphoryl anhydride, ester and thioester. The strong functionalities include, but are not limited to ether, thioether, amine, amide and ester. The use of a strong linker functional group between the spacer group and the biologically-active compound will tend to decrease the rate at which the compound will be released at the target site, whereas the use of a weak linker functional group between the spacer group and the compound may act to facilitate release of the compound at the target site. Enzymatic release is, of course, also possible, but such enzyme-mediated modes of release will not necessarily be correlated with bond strength in such embodiments of the invention. Spacer moieties comprising enzyme active site recognition groups, such as spacer groups comprising peptides having proteolytic cleavage sites therein, are envisioned as being within the scope of the present invention.

The present invention also provides methods and compositions of matter for facilitating the entry of antigenically-active peptides into cells and for delivering such peptides to the appropriate intracellular organelles for immunological processing and antigen presentation. This is achieved by conjugating the desired antigenically-active peptide to a polar lipid carrier and administering this conjugate to an animal by standard techniques.

Experimentally, it was found that fluorescent ceramide is distributed differentially in different cells. These results suggest that by the proper choice of polar lipid conjugate, intracellular targeting of antigenically-active peptides can be achieved. This result enables the delivery of antigenically-active peptides to nascent major histocompatibility complex protein molecules of both types (class I and class II). Thus, antigen presentation of such antigenically-active peptides can be achieved, allowing activation of both humoral and cellular immunity.

The invention specifically provides methods for preparing and administering vaccines against pathological microorganisms, and compositions comprising such vaccines. Vaccines provided by the invention include but are not limited to vaccines against poliovirus, measles virus, rabies virus, the rubella virus, human immunodeficiency virus, Epstein-Barr virus, varicella zoster, herpes simplex virus, hepatitis virus, human papilloma virus, and the microorganisms responsible for diphtheria, malaria, scarlet fever, viral and bacterial pneumonia, whooping cough, scrapie, and other diseases.

Alternatively, pathological conditions (such as autoimmune disease) may be alleviated by the selective blocking of self-antigen presentation by the administration of the appropriate blocking peptides covalently linked to an appropriate polar lipid carrier. Autoimmune diseases intended for this treatment include but are not limited to diabetes type I, lupus erythematosus, rheumatoid arthritis, encephalomyelitis, Hashimoto's disease, oophoritis, orchiditis, myasthenia gravis, polyneuritis, polymyositis, dermatomyositis, scleroderma, rheumatoid carditis, Sjögen's syndrome, and autoimmune hemolytic anemias.

Similarly, tissue and organ transplantation rejection can be inhibited by the selective blocking of nonself-antigen presentation by the administration of the appropriate blocking peptides covalently linked to an appropriate polar lipid carrier. The methods of this invention are intended to be useful in inhibiting rejection of transplanted organs and tissues including kidney, liver, pancreas, lung, heart, cornea, bone marrow, skin, endocrine organs, and portions of the gastrointestinal tract, although this is not intended to be an exhaustive listing of all the uses for this aspect of the invention.

Animals to be treated with polar lipid-antigenically active peptide conjugates using the methods of the invention are intended to include all vertebrate animals, preferrably domesticated animals, such as cattle, horses, goats, sheep, fowl, fish, household pets, and others, as well as wild animals, and most preferably humans.

An antigenically-active peptide, as used herein, is defined as including, but not necessarily limited to any peptide, comprising 4-100 amino acids (including naturally-occurring amino acids, amino acid analogues and derivatives thereof), that is capable of eliciting or inhibiting and immunological response in an animal, preferably a human being. More specifically, such antigenically-active peptides are characterized by their capacity to induce, augment or block (i.e., down-regulate) humoral and/or cellular immune responses *in vivo.* Such peptides include peptides whose amino acid sequence is known and can be chemically synthesized *in vitro,* or produced using recombinant genetic means, as well as mixtures of such peptides. Alternatively, antigenic proteins can be chemically or enzymatically degraded *in vitro,* and mixtures of peptides so produced then used for preparing peptide-polar lipid conjugates of the invention. Covalently-linked multimers of such antigenically-active peptides are also encompassed by the invention.

Representative antigenically-active peptide sequences include but are not limited to:

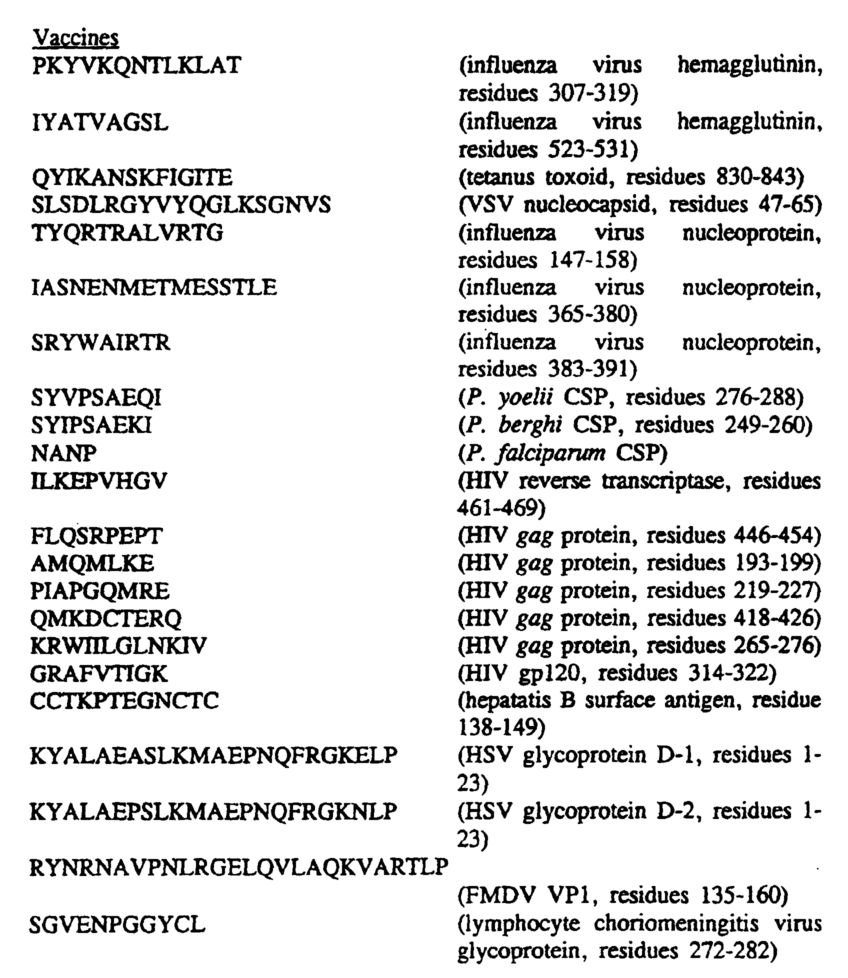

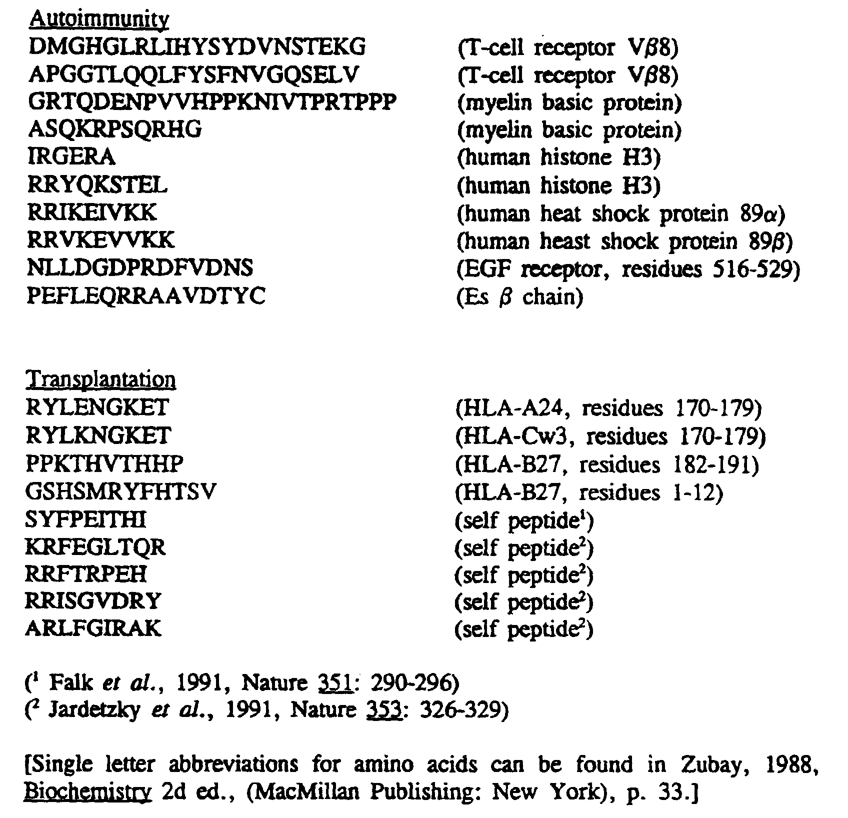

The following Examples illustrate certain aspects of the above-described method and advantageous results. The following examples are shown by way of illustration and not by way of limitation.

### EXAMPLE 1

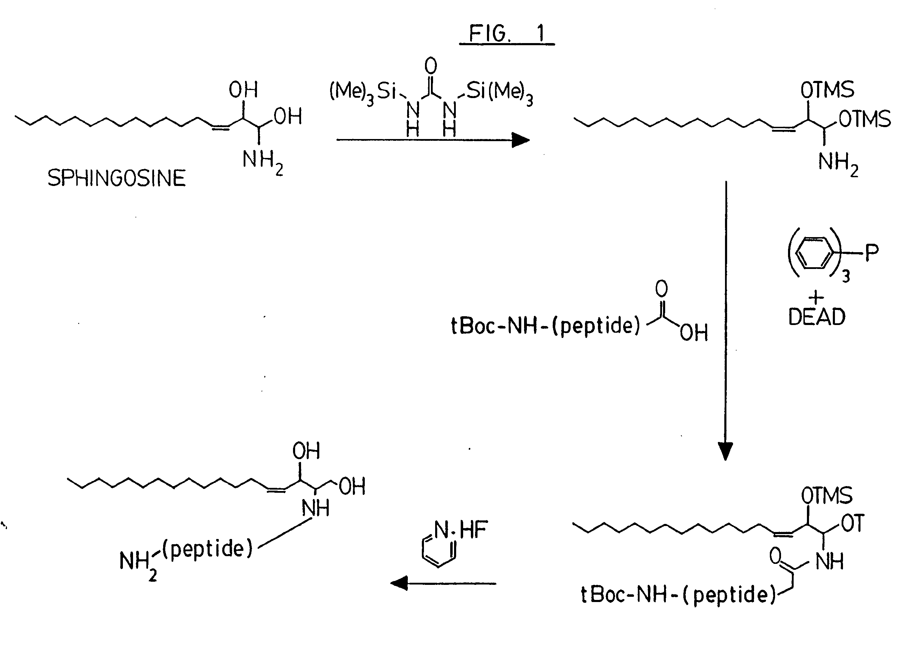

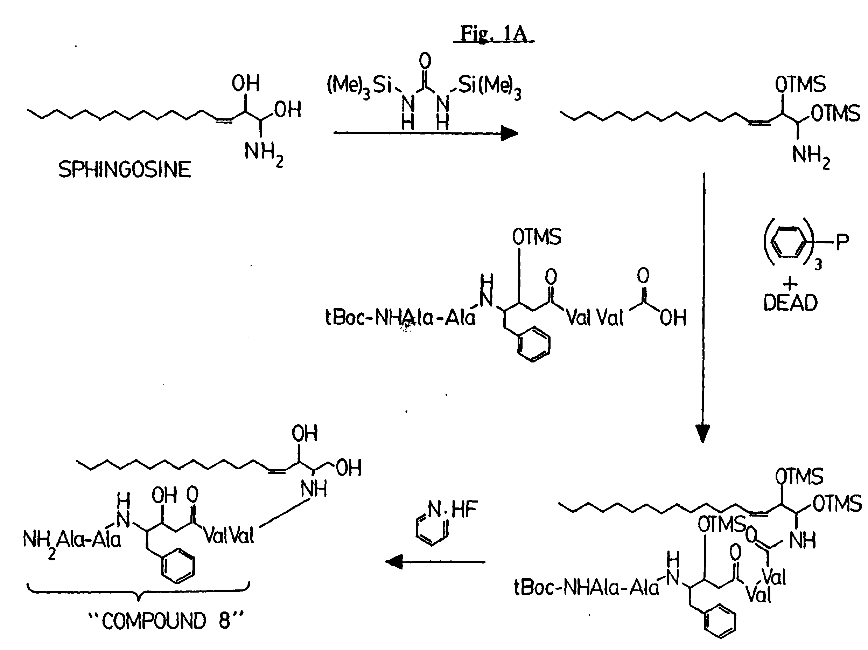

An antigenically-active peptide is conjugated to sphingosine as follows. Sphingosine is reacted with 1,3 *bis*(trimethylsilyl)urea as described by Verbloom *et al.* (1981, Synthesis 1032: 807-809) to give a trimethylsilyl derivative of sphingosine. The sphingosine derivative is then conjugated with the antigenically-active peptide in which the terminal amine and any of the constituent amino acid sidechain reactive amines are covered by *tert-* butoxycarbonyl (*t-*Boc) protecting groups in the presence of diethylazodicarboxylate (DEAD) and triphenyl phosphine as described by Kishimoto (1975, Chem. Phys. Lipids 15: 33-36). The sphingosine/peptide conjugate is then reacted in the presence of pyridine hydrofluoride as described by Matsuura *et al.* (1976, J. Chem. Soc. Chem. Comm. pg. 451-459) to remove the *t-*Boc protecting group, to yield the antigenically-active peptide covalently linked to sphingosine through an amide bond. This reaction scheme is illustrated in Figure 1.

### EXAMPLE 1A

An antiviral compound (HIV1 protease inhibitor; compound 8) is conjugated to sphingosine as described in Example 1 and illustrated in Figure 1A.

### EXAMPLE 2

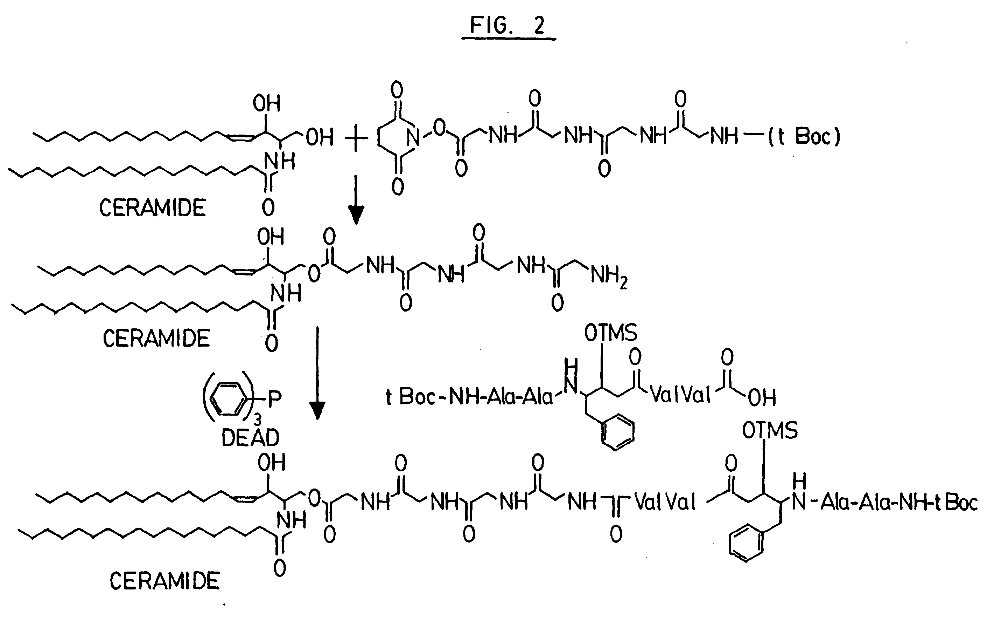

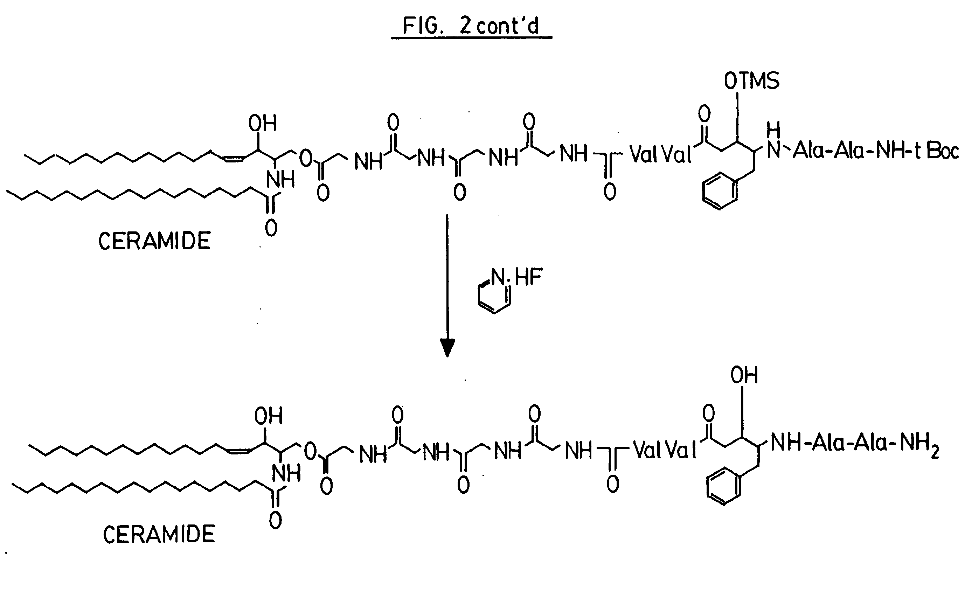

An antiviral compound (compound 8) is conjugated to ceramide *via* a polyglycine spacer as follows and as illustrated in Figure 2A. The amino terminus of polyglycine is protected by a *t-*Boc group. Polyglycine is conjugated through its carboxy terminus to ceramide forming an ester linkage, as described in Anderson *et al., ibid.* The resulting compound is then conjugated through the amino terminus of the polyglycine residue. The amino terminus of Compound 8 is also protected by a *t-*Boc protecting group. Conjugation with polyglycyl-sphingosine takes place between the amino terminus of the polyglycyl spacer moiety and the carboxy terminus of the HIV-1 protease inhibitor. This reaction is carried out in the presence of DEAD and triphenyl phosphine as described in Example 1. Following this conjugation, the amino terminus of the HIV-1 protease inhibitor residue is deprotected according to the method of Matsuura *et al., ibid.*

### EXAMPLE 3

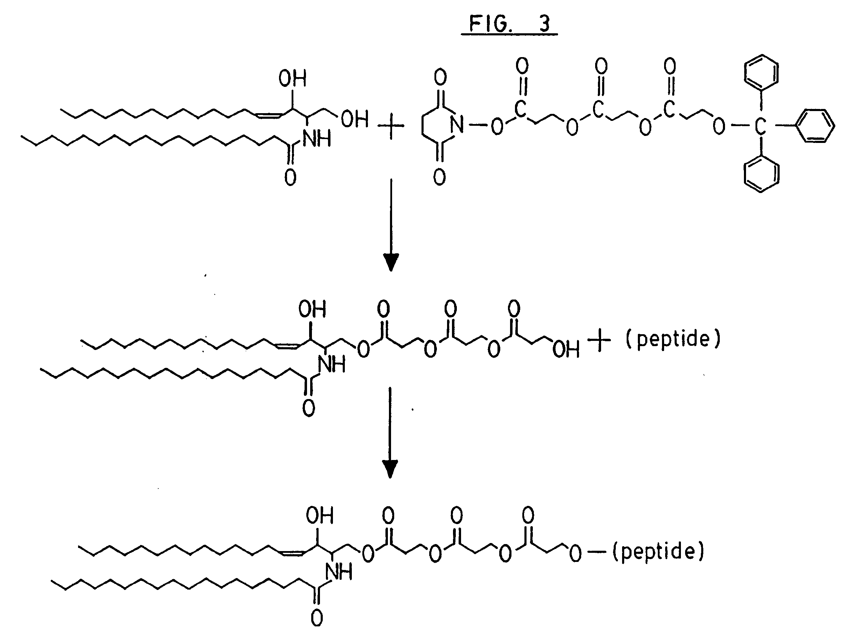

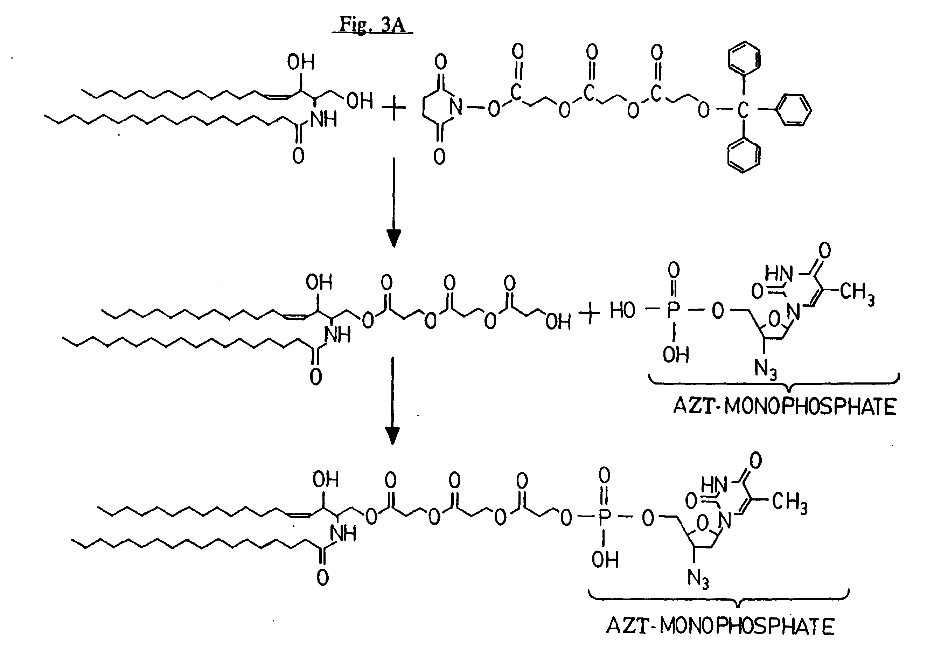

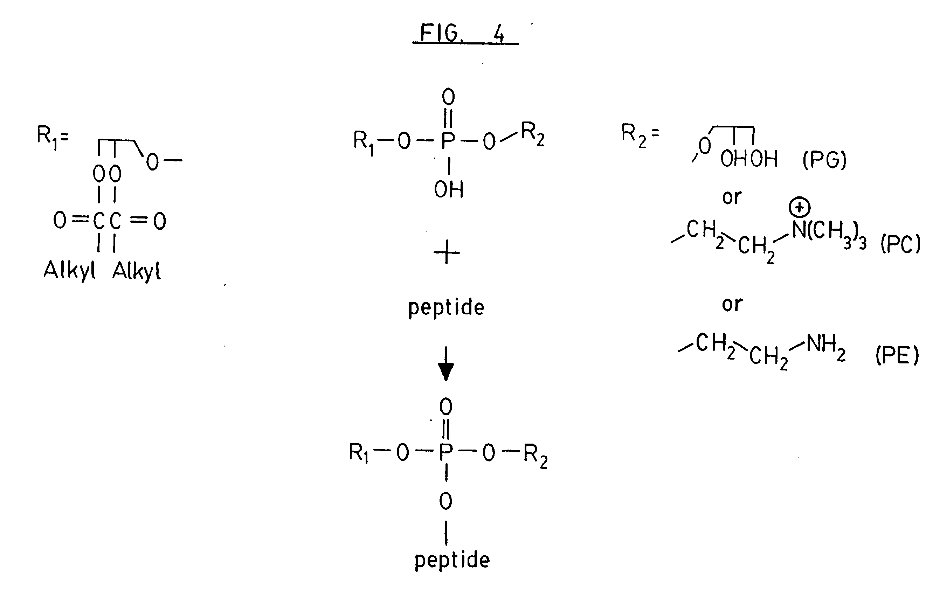

An antigenically-active peptide compound is conjugated to ceramide *via* an oligomeric 3-hydroxy-propanoic acid spacer wherein a first end of the oligomeric spacer is conjugated to ceramide through an ester functional group, and wherein the antigenically-active peptide is conjugated to a second end of the polyester spacer through an amide linkage to the amino terminus of the antigenically-active peptide. The polyester spacer is first obtained, having a carboxyl group at a first end and a triphenylmethyl group esterified to a second end. This spacer is conjugated to ceramide at its first end through an ester functional linker group according to the method of Anderson *et al.* (1963, J. Am. Chem. Soc. 85: 3039). This compound is then conjugated through the second end of the spacer compound to the antigenically-active peptide by means of a amide linkage according to the method of Verbloom *et al.* (1981, Synthesis 1032: 807-809). This reaction scheme is illustrated in Figure 3.

### EXAMPLE 3A

An antiviral compound is prepared wherein ceramide is first conjugated to a first end of an oligomeric 3-hydroxy propanoic acid spacer through an ester functional group, and wherein AZT is conjugated to a second end of said polyester spacer through a phosphodiester bond. First a polyester spacer is obtained, having a carboxyl at a first end and a triphenylmethyl group esterified to a second end. This spacer is conjugated to ceramide at its first end through an ester functional linker group according to the method of Anderson *et al., ibid.* This compound is then conjugated through the second end of the spacer compound to AZT monophosphate by means of a phosphodiester bond according to the method of Baer (1955, Can. J. Biochem. Phys. 34: 288). In this antiviral compound, the bond breakage between the spacer and the drug would be slow in the absence of a phosphohydrolase. This reaction scheme is illustrated in Figure 3A.

### EXAMPLE 4

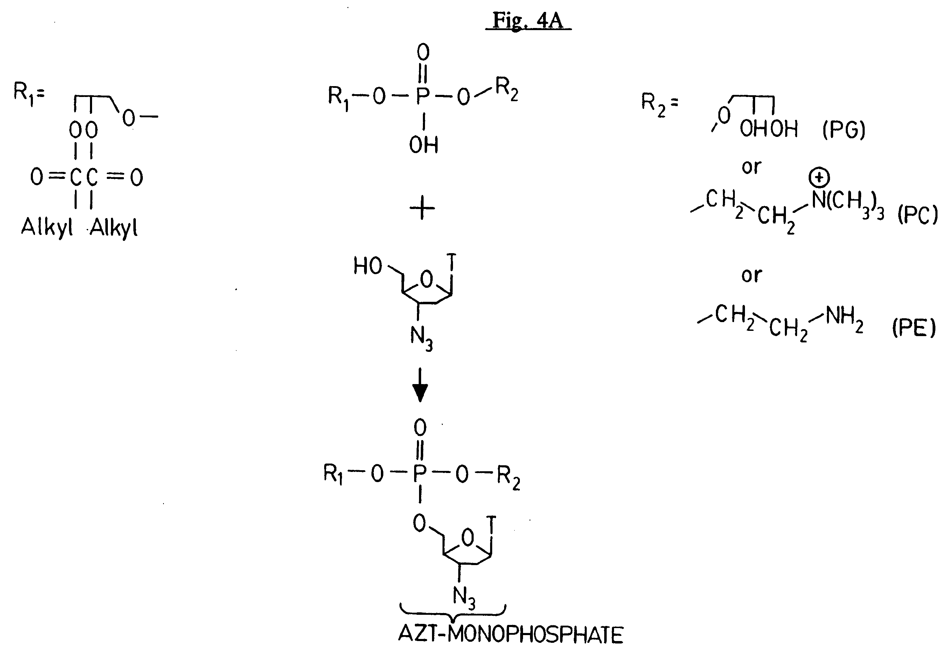

An antigenically-active peptide compound wherein phosphatidic acid, phosphatidyl choline, phosphatidyl serine, phosphatidyl inositol, phosphatidyl glycerol or phosphatidylethanolamine is linked through a phosphoester linker functional group to the antigenically-active peptide. Phosphatidic acid, phosphatidyl choline, phosphatidyl seine, phosphatidyl inositol, phosphatidyl glycerol or phosphatidyl ethanolamine is conjugated to the carboxyl terminus of the antigenically-active peptide according to the method of Salord *et al.* (1986, Biochim. Biophys. Acta 886: 64-75). This reaction scheme is illustrated in Figure 4.

### EXAMPLE 4A

An antiviral compound is prepared as described in Example 4 and illustrated in Figure 4A wherein phosphatidic acid, phosphatidyl choline, phophatidyl glycerol or phosphatidyl ethanolamine is linked through a phosphoester linker functional group to AZT.

### EXAMPLE 5

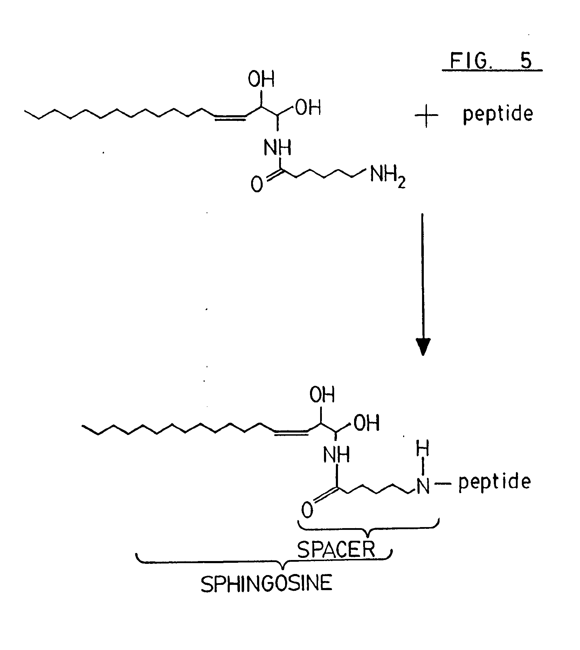

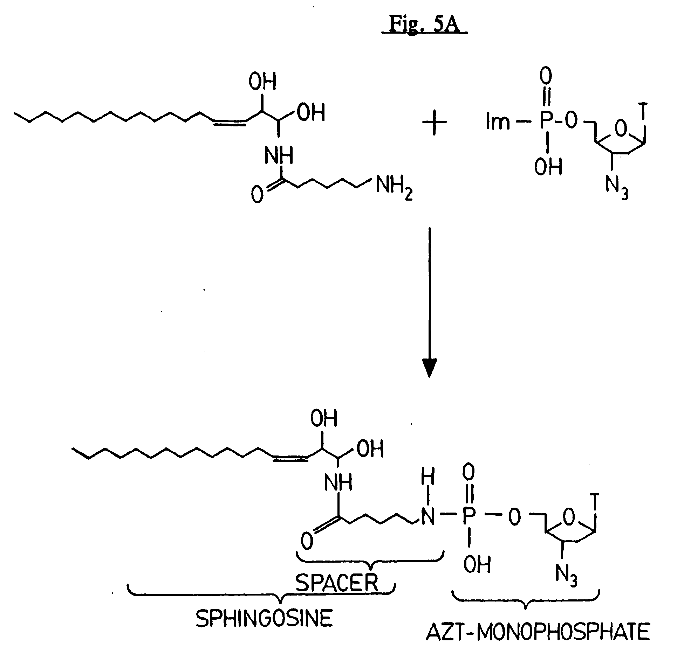

An antigenically-active peptide compound is prepared wherein aminohexanoyl sphingosine is conjugated to the carboxyl terminus of peptide the antigenically-active peptide. Aminohexanoyl sphingosine is conjugated with the antigenically-active peptide according to the method of Kishimoto (1975, Chem. Phys. Lipid 15: 33-36). This reaction scheme is illustrated in Figure 5.

### EXAMPLE 5A

An antiviral compound is prepared wherein aminohexanoyl sphingosine is conjugated to AZT. Aminohexanoyl sphingosine is conjugated with AZT monophosphoimidazole as described in Figure 5 and illustrated in Figure 5A to yield aminohexanoyl sphingosine conjugated to AZT through a phosphoramide bond.

### EXAMPLE 6

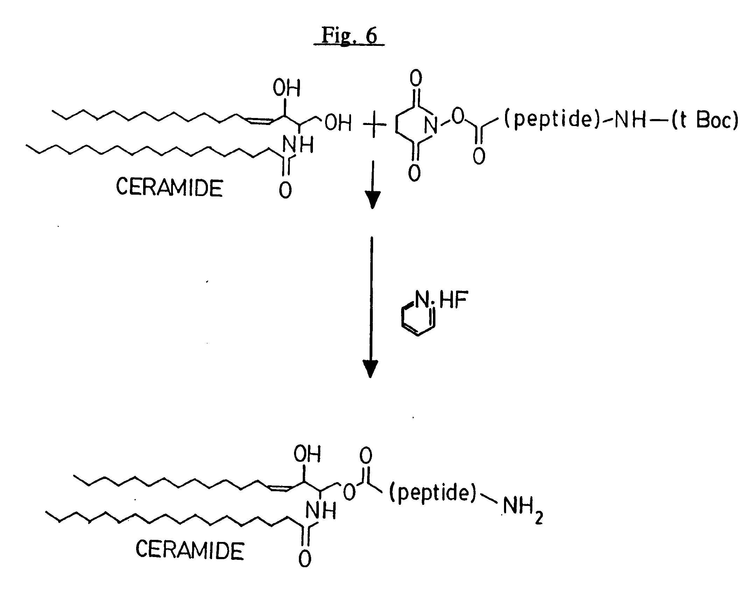

An antigenically-active compound is prepared consisting of ceramide conjugated to a first end of an antigenically-active peptide through an ester linker functional group or an amide linker functional group. The antigenically-active peptide has both a carboxyl terminus and an amino terminus, the amino terminus being protected by a *t-*Boc group. The antigenically-active peptide is conjugated through its carboxyl terminus to ceramide forming an ester linkage, as described by Anderson *et al.* (1963, J. Chem. Soc. Chem. Comm. 85: 3039). The amino terminus of the antigenically-active peptide is then deprotected according to the method of Matsuura *et al.* (1976, J. Chem. Soc. Chem. Comm. pg. 451). This reaction scheme is illustrated in Figure 6.

### EXAMPLE 7

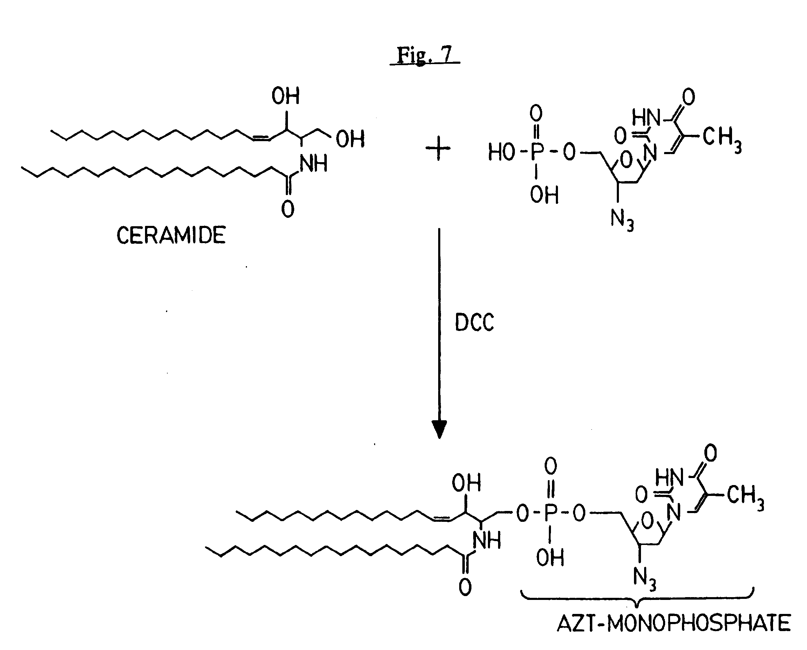

An antiviral compound consisting of ceramide conjugated to AZT-monophosphate is provided. Ceramide is reacted with AZT-monophosphate in the presence of dicyclohexylcarbodiimide as described in Smith and Khorana (1958, J. Amer. Chem. Soc. 80: 1141) to yield ceramide conjugated through a phosphodiester bond to AZT-monophosphate. This reaction scheme is illustrated in Figure 7.

### EXAMPLE 8

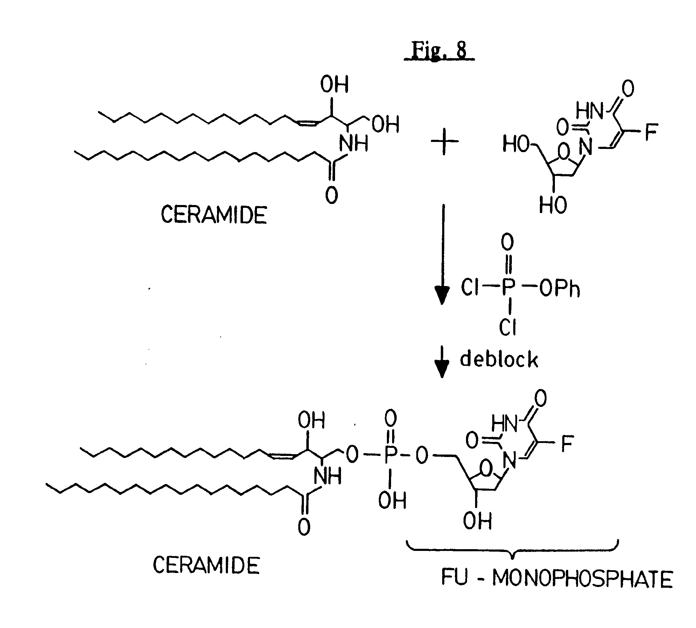

An antineoplastic compound is prepared wherein sphingosine is conjugated through a phosphodiester bond to 5-fluorodeoxyuridine. Sphingosine is reacted with 5-fluorodeoxyuridine in the presence of dichlorophenyl phosphate according to the method of Baer, *ibid.,* to yield sphingosine conjugated to 5-fluorodeoxyuridine through a phosphodiester bond. This reaction scheme is illustrated in Figure 8.

### EXAMPLE 9

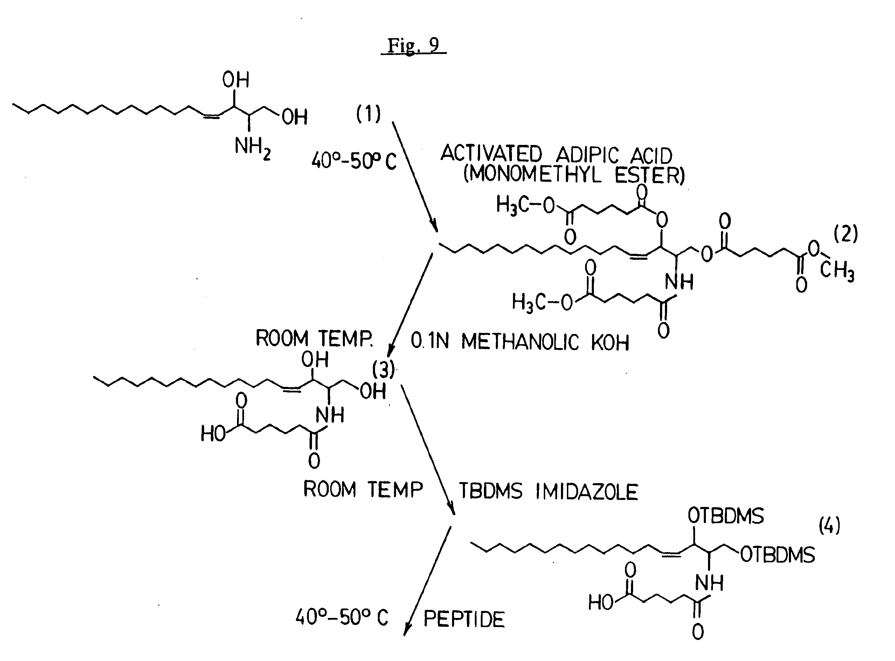

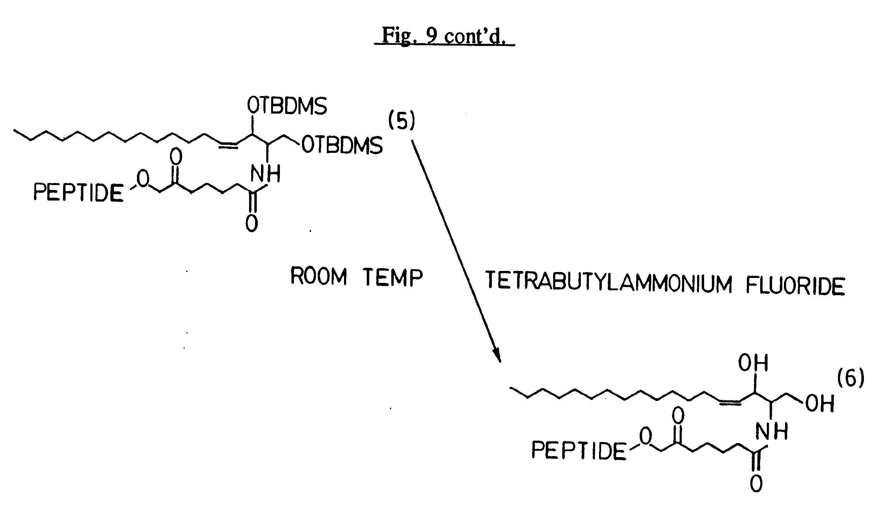

An antigenically-active peptide is prepared wherein sphingosine is conjugated to the amino terminus of the antigenically-active peptide through an adipic acid spacer. The primary amino and hydroxyl groups of sphingosine are acylated by reaction with adipic acid monomethyl ester overnight at 40-50°C, followed by base hydrolysis of the ester (in 0.1N methanolic KOH). The free hydroxyl group of this intermediate is protected using *t-*butyldimethylsilane (TBDMS) by reaction overnight at room temperature. The antigenically-active peptide is then covalently linked to the free carboxyl end of the adipic acid spacer activated by reaction overnight at 40-50°C in the presence of carbonyl diimidazole (the details of this reaction may be found in *Enzyme,* vol.18, p. 310, 1974). The TBDMS protecting groups are then removed using tetrabutylammoniumfluoride to yield the antigenically-active peptide product. This reaction scheme is illustrated in Figure 9.

### EXAMPLE 10

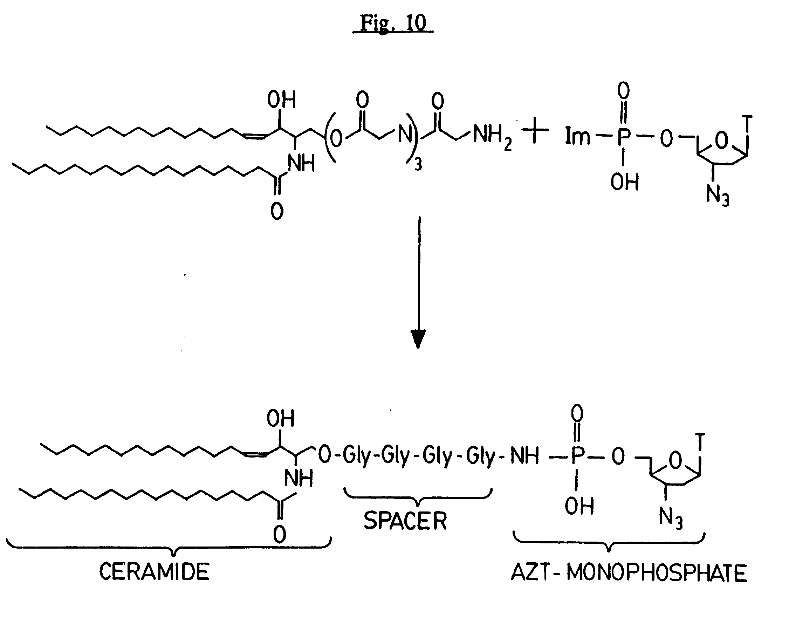

An antiviral compound is prepared wherein ceramide is conjugated through an ester functional group to a first end of a polyglycine spacer, and wherein AZT is conjugated through a phosphoester functional group to a second end of the polyglycine spacer. Ceramide is first conjugated through an ester functional group to a first end of a polyglycine spacer (as described in Example 2). The ceramide-polyglycine compound is then conjugated through a phosphoester bond to a second end of the polyglycine spacer to AZT monophosphate according to the method of Paul and Anderson, *ibid.* This reaction scheme is illustrated in Figure 10.

### EXAMPLE 11

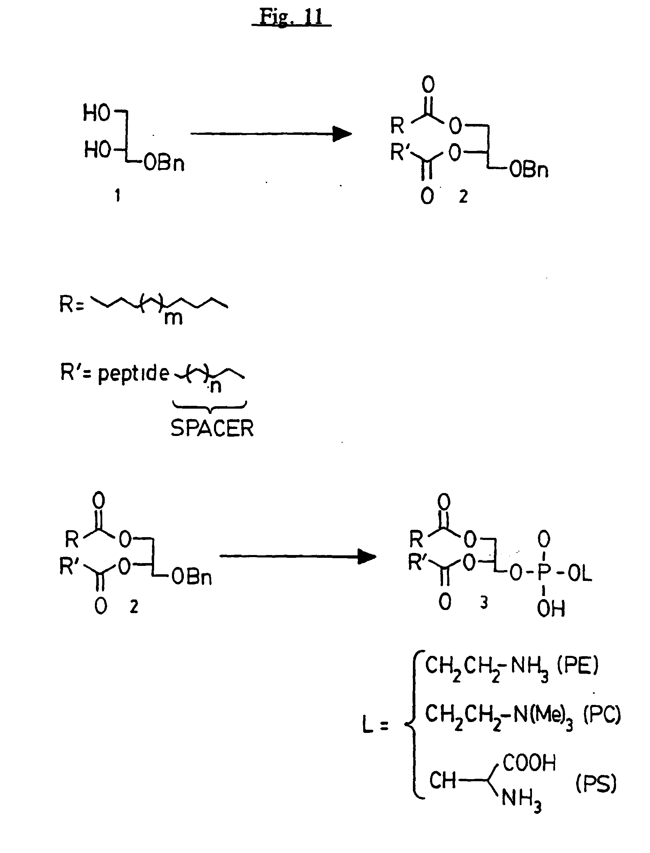

An antigenically-active peptide compound is prepared wherein phosphatidic acid, phosphatidyl choline, phosphatidyl glycerol or phosphatidyl ethanolamine is linked through the *sn*-2 or *sn*-1 hydroxyl of the lysophospholipid to the antigenically-active peptide using the methods of Martin & Josey (1988, Tetrahedron Lett. 29: 3631-3634). This reaction scheme is illustrated in Figure 11. Briefly, the antigenically active peptide (whether or not covalently linked to a spacer moiety), or alternatively a fatty acid, is conjugated to the *sn*-1 hydroxyl of 3-*sn*-benzyl glycerol by reaction at 0°C in the presence of dimethylaminopyridine/ dicyclohexylcarbodiimide/ methylene chloride (DMAP/DCC/CH₂Cl₂). A fatty acid, or alternatively the antigenically-active peptide (whether or not covalently linked to a spacer moiety), is then conjugated to the *sn*-2 hydroxyl of 3-*sn*- benzyl-1-*sn*-substituted glycerol at 20°C in the presence of DMAP/DCC/CH₂Cl₂. The 3-*sn* position is then deprotected at 45°C in ethanol/acetic acid in the presence of platinum black and H₂. The appropriate polar head group is then phospho-esterified to the *sn*-3 position in the presence of phenyldichlorophosphite/ diisopropylethylamine/ tetrahydrofuran at -78°C.

Alternatively, the antigenically-active peptide (whether or not covalently linked to a spacer moiety), can be conjugated to phospholipid following enzymatic deacylation of a diacylphospholipid with phospholipase A₂ using the method of Eibi *et al.* (1983, Meth. Enzymol. 98: 623).

### EXAMPLE 12

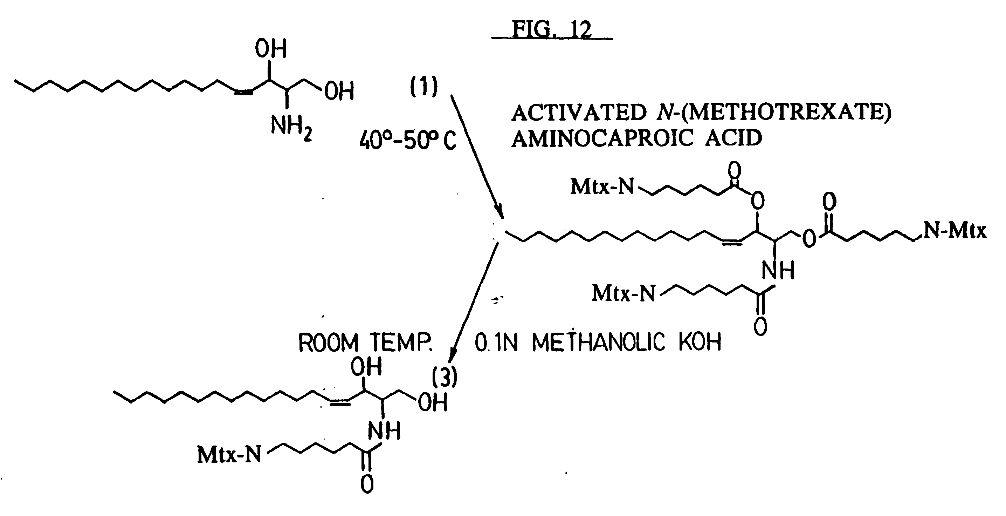

An antigenically-active peptide is prepared wherein the anti-neoplastic agent methotrexate (Mtx) is conjugated to sphingosine *via* a 6-aminocaproic acid spacer. This reaction scheme is illustrated in Figure 12. The primary amino and hydroxyl groups of sphingosine are acylated by reaction with activated *N*-(methotrexate)aminocaproic acid overnight at 40-50°C, followed by base hydrolysis in 0.1N methanolic KOH. The Mtx derivative of 6-aminocaproic acid is synthesized by activating the carboxylic acid moiety of Mtx and reacting with 6-aminocaproic acid for 2 days at 60-70°C. This reaction is stopped under acidic conditions to liberate anhydrides that form under these conditions.

### EXAMPLE 13

Antigenically-active peptide-polar lipid conjugates of the invention are used as follows. For use as a vaccine, the conjugate, the naked peptide and a negative control (saline) are administered to an animal using both optimal and suboptimal dosages and the most appropriate route of administration. After an optimal time period (determined from the nature of the immunological response to be elicited), both sera and lymphoid cells are collected from the animal and tested for reactivity to the antigen. Lymphoid cells are isolated using conventional methods (Ficoll-Hypaque density gradient centrifugation) and tested for cytotoxic activity against control autologous macrophage/monocyte preparations exposed to and subsequently presenting the original peptide antigen. Testing is performed using the ⁵¹Cr release assay of Malkovsky *et al.* (1982, Nature 300: 652-655). Antibody response is tested using standard radioimmunoassay methods (*see,* Brenner *et al.,* 1984, Eur. J. Immunol. 14: 1021-1027). Briefly, Linbro flexible plates are coated with the specific peptide antigen by overnight incubation of a peptide solution (1 mg/mL) in phosphate buffered saline (PBS). Nonspecific binding is then blocked by treatment of the plates with a solution of 0.2% bovine serum albumin and 0.2% gelatin in PBS. The plates are then washed, sera to be tested is then added, and the plates rewashed after incubation of the sera on the plates. ¹²⁵I-labeled anti-IgG and anti-IgM antibodies are then added, the plates washed and bound radioactivity counted. The amount of peptide-antigen specific antibody present on each plate is then calculated relative to a standard curve prepared with known amounts of anti-peptide antibody. From these experiments the titre of specific antibody against each peptide antigen is calculated for each experimental sera tested.

For use in preventing transplant rejection or to treat autoimmune disease, the appropriate administration protocol is determined by vaccination of an animal as described above. After an empirically-determined optimal time period (determined from the nature of the immunological response to be elicited), both sera and lymphoid cells are collected from the animal and tested for reactivity to either self-antigen (for autoimmune disease uses) or heterologous transplantation antigens, as described above.

It should be understood that the foregoing disclosure emphasizes certain specific embodiments of the invention and that all modifications or alternatives equivalent thereto are within the spirit and scope of the invention as set forth in the appended claims.

## Claims

1. A composition of matter for intracellular targeting of a biologically-active compound comprising a biologically-active compound having a molecular weight of less than about 1500 daltons, a polar lipid carrier, first and second linker functional groups and an organic spacer, characterised in that the spacer has a first end and a second end and wherein the polar lipid is attached at a site within the hydrophobic tail of the polar lipid to the first end of the spacer through the first linker functional group and the biologically-active compound is attached to the second end of the spacer through the second linker functional group.

2. A composition as claimed in claim 1, wherein the spacer allows the biologically-active compound, peptide or drug to act without being released at an intracellular site and wherein the first linker functional group attached to the first end of the spacer is strong and the second linker functional group attached to the second end of the spacer is weak.

3. A composition as claimed in any one of claims 1 to 3, wherein the spacer allows the facilitated hydrolytic release or facilitated enzymatic release of the biologically-active compound, peptide or drug at an intracellular site and wherein the first linker functional group attached to the first end of the spacer is strong and the second linker functional group attached to the second end of the spacer is weak.

4. A composition as claimed in any preceding claim, wherein the spacer is a peptide of formula (amino acid)ₙ, n being an integer between 2 and 25 and the peptide being a polymer of a particular amino acid.

5. A composition of matter for intracellular targeting of a biologically-active compound comprising a biologically-active compound having a molecular weight of less than about 1500 daltons and having a first functional linker group, and a polar lipid carrier having a second functional linker group, characterised in that the compound is covalently linked to the polar lipid carrier at a site within the hydrophobic tail of the polar lipid by a chemical bond between the first and second functional linker groups.

6. A composition as claimed in claim 5, wherein the first functional linker group is a hydroxyl group, a primary or secondary amino group, a phosphate group or substituted derivatives thereof or a carboxylic acid group.

7. A composition as claimed in claim 5 or claim 6, wherein the second functional linker group is a hydroxyl group, a primary or secondary amino group, a phosphate group or substituted derivatives thereof or a carboxylic acid group.

8. A composition as claimed in any preceding claim, wherein the polar lipid is sphingosine, ceramide, phosphatidyl choline, phosphatidyl glycerol, phosphatidyl ethanolamine, phosphatidyl inositol, phosphatidyl serine, cardiolipin and phosphatidic acid.

9. A composition as claimed in any preceding claim, wherein the biologically-active compound is a peptide.

10. A composition as claimed in claim 9, wherein the peptide is an antigenically active peptide.

11. A composition as claimed in any preceding claim, wherein the biologically-active compound is a drug.

12. A composition as claimed in claim 11, wherein the drug is an antiviral or antineoplastic drug.

13. A composition as claimed in claim 1, being 3'-azido-3'-deoxythymidine monophosphate-[tri-β-hydroxypropionylester]-O^{x}-ceramide ester, N-[1-(3'-azido3'-deoxythymidinemonophosphate) aminohexanoyl]sphingosine amide, 3'-azido-3'deoxythymidine monophosphate-O^{x}-ceramide ester, 5-fluorouridine deoxyribosemonophosphate-O^{x}-ceramide ester, phosphatidylcholine-3'-azido-3'-deoxythymidine monophosphate, phosphatidylglycerol-3'-azido-3'-deoxythymidine monophosphate, phosphatidylethanolamine-3'-azido-3'-deoxythymidine monophosphate, N-methotrexate ceramide, 1-phenyl-2-(alanylalanyl) amino-3-hydroxyl-pentanoyl-valinylvalinylglycylglycylglycylgylcyl ceramide ester, 3'-azido-3'-deoxythymidine monophosphate-glycylglycylglycylgylcyl ceramide ester or [1-phenyl-2-(alanylalanyl) amino-3-hydroxyl-pentanoyl] valinylvalinyl sphingosine amide.

14. The use of a composition as claimed in claim 10 for the manufacture of a medicament for eliciting an immune response in an animal against a pathogenic microorganism, characterised in that the medicament comprises a pharmaceutically acceptable carrier and an amount of the said composition sufficient to elicit an immunological response in the animal.

15. The use of a composition as claimed in claim 10 for the manufacture of a medicament for alleviating an autoimmune disease in an animal, characterised in that the medicament comprises a pharmaceutically acceptable carrier and an amount of the said composition sufficient to inhibit the autoimmune response in the animal.

16. The use of a composition as claimed in claim 10 for the manufacture of a medicament for preventing or suppressing tissue or organ transplant rejection in an animal, characterised in that the medicament comprises a pharmaceutically acceptable carrier and an amount of the said composition sufficient to inhibit transplant rejection in the animal.

17. The use of a composition as claimed in claim 12 for the manufacture of a medicament for administering an antiviral or antineoplastic drug to an animal, characterised in that the medicament comprises a pharmaceutically acceptable carrier and an amount of the said composition sufficient to elicit a therapeutic effect in the animal.

18. The use of a composition as claimed in claim 1 for the manufacture of a medicament for administering a biologically-active compound to an animal, characterised in that the medicament comprises a pharmaceutically acceptable carrier and an amount of the said composition sufficient to elicit a therapeutic effect in the animal.

19. The use of a composition as claimed in claim 5 for the manufacture of a medicament for administering a biologically-active compound to an animal, characterised in that the medicament comprises a pharmaceutically acceptable carrier and an amount of the said composition sufficient to elicit a therapeutic effect in the animal.

20. A composition as claimed in any of claims 1 to 13 for use as a pharmaceutical.

21. A use claimed in any of claims 14 to 19, wherein the animal is a human.
